(19) Europäisches Patentamt
European Patent Office
Office européen des brevets

(11) Publication number: **0 088 494**
**B1**

(12) **EUROPEAN PATENT SPECIFICATION**

(45) Date of publication of patent specification: **02.05.85**

(51) Int. Cl.$^4$: **C 07 C 1/20**, C 07 C 1/24, C 07 C 11/02, B 01 J 29/28

(21) Application number: **83300359.3**

(22) Date of filing: **25.01.83**

(54) **Process for converting methanol into olefins.**

(30) Priority: **05.02.82 US 345985**
**05.02.82 US 346426**
**05.02.82 US 345984**

(43) Date of publication of application:
**14.09.83 Bulletin 83/37**

(45) Publication of the grant of the patent:
**02.05.85 Bulletin 85/18**

(84) Designated Contracting States:
**BE DE FR GB IT NL**

(56) References cited:
**US-A-4 062 905**
**US-A-4 229 608**
**US-A-4 251 484**
**US-A-4 338 475**

(73) Proprietor: **MOBIL OIL CORPORATION**
**150 East 42nd Street**
**New York New York 10017 (US)**

(72) Inventor: **Lee, Wooyoung**
**4 Cohasset Lane**
**Cherry Hill New Jersey 08003 (US)**
Inventor: **Sapre, Ajit Vishwanath**
**3410 Clubhouse Drive**
**West Deptford New Jersey 08066 (US)**
Inventor: **Yurchak, Sergei**
**211 Timber Jump Lane**
**Media Pennsylvania 19063 (US)**

(74) Representative: **West, Alan Harry et al**
**Mobil Court 3 Clements Inn**
**London WC2A 2EB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 088 494 B1**

Courier Press, Leamington Spa, England.

**Description**

This invention relates to the production of olefins from methanol and more particularly, to the catalytic conversion of methanol into ethylene.

The ever-increasing demand for olefinic feedstocks has periodically caused a shortage of petrochemical raw materials either because of a limitation in availability of suitable quality petroleum feedstocks from which they have traditionally been derived or a limitation in naphtha cracking capacity. An alternative source of ethylene from non-petroleum sources is one obvious means of keeping pace with the demand for ethylene and other olefins.

It is known that methanol and/or dimethyl ether, which may be obtained from coal, natural gas or biomass, can be converted into more complex hydrocarbons such as olefins and aromatics, by utilizing a group of catalytic zeolites exemplified by ZSM-5 zeolite. Ethylene is one of the olefinic hydrocarbons which may be obtained from such catalytic conversions. The reaction is highly exothermic and the olefins initially formed have a tendency to undergo further reaction to produce aromatic hydrocarbons useful in the production of motor gasoline.

Many patent publications are concerned with various aspects of the conversion of methanol and/or dimethyl ether into light olefins. Thus, the production of olefins from aliphatic ethers by catalytic conversion with a HZSM-5 zeolite is described in U.S. Patent 3,894,106. U.S. Patent 3,979,472 describes the conversion of lower alcohols and their ethers with a composite of antimony oxide and a ZSM-5 zeolite to produce a mixture of ethylene, propylene and mononuclear aromatics. U.S. Patent 4,025,572 discloses that ethylene selectivity can be improved by combining ZSM-5 with an inert diluent while a similar result is achieved, according to U.S. Patent 4,025,575, through use of subatmospheric partial pressure of the feed. Selectivity to ethylene is also improved by employing ZSM-5 zeolite in large crystal form of at least about 1 micron either alone (U.S. Patent 4,025,571) or in combination with added metals (U.S. Patent 4,148,835). Better selectivity is obtained also by interdispersing amorphous silica within the interior of the crystalline structure of the zeolite catalyst (U.S. Patents 4,060,568 and 4,100,219).

Although such processes perform exceptionally well and are unusually effective in converting lower aliphatic alcohols into olefinic hydrocarbons, it has been found that these conversions are exothermic to varying degrees depending on the particular reactant. For example, the amount of heat generated in the conversion of lower alcohols into hydrocarbon product may be estimated to be in the following ranges:

| Alcohol reactant | Heat produced, kJ per kg of hydrocarbon product |
|---|---|
| Methanol | 2300—4600 |
| Ethanol | 460—1450 |
| Propanol | 35—840 |

While it is desirable for a reaction to be exothermic, since this obviates the need for an external source of heat to drive the reaction, large heat generation loads can require substantial investment in complex reactors with extensive internal cooling systems. It can be seen from the table above that the conversion of methanol, and to a lesser degree of ethanol, could be considered excessively exothermic in this regard. Furthermore, because of the inherent character and efficiency of the crystalline aluminosilicate zeolite catalysts, the reaction of methanol, and to a lesser degree of ethanol, tends to be self-accelerating, creating excessively hot local regions in the catalyst bed where the reaction tends to go to completion. In an adiabatic catalyst bed reactor, these highly exothermic reactions can result in high catalyst aging rates, and may cause thermal damage to the catalyst. Furthermore, such high temperatures can cause an undesirable product distribution. Therefore, it is critical in the conversion of methanol into useful products to provide sufficient heat dissipation so that temperatures encountered in any portion of the catalyst bed are restricted within predetermined limits.

Additionally, it is generally good engineering practice to conduct reactant conversions at elevated pressures to utilize more effectively the reactor volume and attendant equipment. With a methanol charge, however, elevated pressures tend to produce increased quantities of 1,2,4,5-tetramethylbenzene (durene), an undesirable by-product, while lower pressures, for example less than 450 kPa favor the production of light olefins.

Various techniques have been employed to control the heat released in such processes: see, for example U.S. Patents 3,931,349 (use of light hydrocarbon diluents), 4,052,479 (operating conditions selected to restrict feed conversion to 5—25%) and 4,238,631 (riser reactor and dense fluid catalyst bed).

U.S. Patent 4,035,430 describes a system for controlling the heat release when converting methanol into gasoline boiling-range products. After the feed is converted into an equilibrium mixture in a bed of dehydration catalyst, it is passed through a series of zeolite catalyst beds of increasing size with interstage

quenching with methanol, dimethyl ether and/or light hydrocarbons to remove the heat of reaction. The temperature rise in any one of the beds does not exceed about 28°C and the overall temperature rise does not exceed about 110°C. No detail is provided regarding if or how the relative size of the several catalyst beds influences the thermal stability of the reaction. Further, there is no suggestion that the procedures may be successfully employed where the alcohol feed is to be converted into predominantly olefinic hydrocarbons as opposed to aromatic compounds.

In U.S. Patent 2,319,620, a plurality of fixed beds of catalyst is employed for the endothermic catalytic cracking of hydrocarbons and for the periodic exothermic regeneration of the coked catalyst. The hydrocarbon reactants are divided into a plurality of streams equal to the number of fixed catalyst beds and each stream is passed through only one bed. Flow through each bed may be either axial or radially inward. U.S. Patent 2,475,855 also employs a series of fixed catalyst beds for the endothermic cracking of hydrocarbons. Here the hydrocarbon stream flows sequentially through the series of beds in a radial flow pattern. The radial flow may be inward or outward. The coked catalyst is regenerated *in situ* and the heat generated during regeneration is absorbed within each bed for release during the endothermic cracking of the hydrocarbons. Since the stored heat provides an increase in the temperature of the reactants passing through the bed, additional quantities of liquid hydrocarbons are introduced between the beds through spray nozzles to reduce the temperature of the mixture passing to the next bed. The size of the catalyst beds is increased in the direction of flow to compensate for the introduction of additional reactants between the beds. Neither patent suggests that the procedure it describes can be usefully adapted to an exothermic reaction or that the radial flow pattern employed will provide lower pressure losses than other flow patterns.

Although the effective use of processing equipment is usually dictated by the use of elevated pressures in the conversion of alcohols into olefins, lower pressures produce significantly more light olefins in the product. It is therefore desirable to limit the pressure loss through the equipment so as to permit the use of lower feed inlet pressures.

Furthermore, while it is recognized that olefin selectivity is enhanced by only partial conversion of the alcohol or ether feed, it is also well recognized that such an expedient entails a large economic penalty because the cost of for example methanol dictates that the unconverted reactant must be recovered and recycled.

The present invention is based on the observation that the heat generated in the conversion of methanol alone or in combination with its corresponding ether in the presence of zeolite catalysts can be effectively removed to provide a stable operation by arranging the zeolite catalyst in a series of beds and providing interstage cooling of the effluent from each bed to limit the temperature rise across each bed. It has also been found that the pressure drop across each catalyst bed can be minimized by passing the reaction mixture radially through each of the beds. Moreover, it has been found also that significant improvements in process economics may be achieved by recycling the unconverted alcohol to the dehydration stage and recycling the unconverted ether to the conversion stage.

According to the present invention, there is provided a process for converting methanol into an olefinic hydrocarbon product which comprises the steps of

contacting a feed comprising methanol at an elevated temperature with a dehydration catalyst to obtain an ether-rich product, and

contacting the ether-rich product at an elevated temperature with a catalyst comprising a crystalline aluminosilicate zeolite having either (i) a pore size greater than 5 Angstroms, a silica to alumina mole ratio of at least 12 and a constraint index from 1 to 12 or (ii) pores the major dimension of which is less than 6 Angstroms and pore windows of a size provided by 8-membered rings of oxygen atoms, to achieve a predetermined degree of conversion into olefinic hydrocarbon products, by passing the ether-rich product through a plurality of alternate beds of zeolite catalyst and cooling zones, the reduction in temperature of the reaction mixture by passage through each cooling zone being substantially equal to the increase in temperature in the immediately preceding catalyst bed, the increase in temperature of the reaction mixture by passage through each catalyst bed being no greater than the sensitivity parameter for the conversion of methanol into ethylene and the number of catalyst beds being at least equal to the ratio of the total adiabatic temperature increase at the predetermined conversion to the sensitivity parameter.

The ether-rich product may be passed through the zeolite catalyst beds in either an axial or radial configuration, and the zeolite catalyst beds may be either fixed beds or moving beds. In the case of moving catalyst beds, the flow of reaction mixture through the beds may be either cocurrent or countercurrent to the flow of catalyst.

The olefinic hydrocarbon product of the process will always contain a proportion of unreacted methanol and/or ether; such unreacted materials are suitably separated from the product mixture and recycled to the process, the methanol being recycled to the dehydration stage and the ether being recycled to the conversion stage.

According to the present invention methanol alone or together with its corresponding ether, is converted into olefinic hydrocarbons, particularly ethylene, in a reactor system designed to achieve effective control of the heat released during the reaction, particularly during the conversion of the ether-rich intermediate product into olefinic hydrocarbons. Preferably, the reactants comprise methanol and dimethyl ether and the olefinic product is predominantly ethylene.

Although the process of the invention concerns the conversion of methanol into an olefinic product, the feedstream to the process may include minor amounts of other alcohols, for example ethanol, propanol and isopropanol.

In the first stage of the process, the methanol is contacted with a dehydration catalyst to produce water and an ether-rich intermediate product. The dehydration catalyst may be any catalyst which results in the intermolecular dehydration of the alcohol reactant to form an ether-rich product of higher carbon to oxygen ratio than the feed.

The dehydration reactions that can take place include those that form simple and mixed ethers such as dimethyl ether and diethyl ether. These intermediates may be formed by the intermolecular dehydration of corresponding alcohol reactants, and all of these condensations are exothermic. While this dehydration reaction by itself, is generally known with alumina compositions, such as gamma alumina, other acidic catalysts known in the art are very effective also for dehydration.

It will be recognized that with a methanol feed, no intramolecular dehydration is possible, and that therefore the dehydration reaction can only proceed exothermally to form dimethyl ether.

The process of the invention comprises two sequential stages of catalytic contact in which both stages generate heat. In the first stage heat generation is limited by restricting the conversion of methanol into approximately an equilibrium mixture comprising dimethyl ether, methanol and water. The conversion product or first stage effluent, because of the generated heat, has a temperature of about 310 to about 400°C, is suitably adjusted to a temperature of about 270 to about 430°C depending on the nature of the second stage zeolite catalyst, by passing it in indirect heat exchange with a circulating heat exchange fluid. For example, the heat exchange fluid may be water or the methanol reactant passed to the first stage.

The second stage catalytic conversion converts the first stage effluent comprising methanol, dimethyl ether and water into an olefin-rich product. The operation is highly exothermic and occurs rapidly in the presence of certain crystalline zeolites and particularly ZSM-5 type crystalline zeolites and small pore crystalline zeolites.

In general, the ZSM-5 type zeolites used in accordance with the invention are crystalline zeolites having a silica/alumina ratio greater than 12 and a Constraint Index (C.I.) between about 1 and about 12. These zeolites and their use as conversion catalysts for lower aliphatic alcohols are described in U.S. patents referred to above, particularly U.S. Patents 3,894,106, 4,025,571, 4,058,576 and 4,148,835.

The preferred zeolites are ZSM-5 type zeolites as exemplified by ZSM-5, ZSM-11, ZSM-12, ZSM-23, ZSM-35 and ZSM-38, ZSM-5 being particularly preferred.

ZSM-5 is described in U.S. Patent 3,702,886; ZSM-11 is described in U.S. Patent 3,709,979; ZSM-12 is described in U.S. Patent 3,832,449; ZSM-23 is described in U.S. Patent 4,076,842; ZSM-35 is described in U.S. Patent 4,016,245; and ZSM-38 is described in U.S. Patent 4,046,859.

Particularly preferred catalysts are those comprising ZSM-5 type zeolite with large crystals i.e. a crystal size of at least 1 micron, as described in U.S. Patents 4,025,571 and 4,148,835. Another class of particularly preferred catalysts are those comprising ZSM-5 and which contain additional ingredients to improve ethylene selectively, such as amorphous silica interdispersed within the interior of the zeolite crystalline structure. Catalysts of this type are described in U.S. Patents 4,060,568 and 4,100,219.

In addition to the ZSM-5 zeolites, other zeolites known in the art as small pore crystalline aluminosilicate zeolites may be employed in accordance with the invention. These small pore zeolites may be either naturally occurring or synthetic and include, for example, erionite, chabazite, zeolite T, zeolite ZK-5 and ZSM-34. Zeolite T is described in U.S. Patent 2,950,952, zeolite ZK-5 in U.S. Patent 3,427,195, and ZSM-34 in U.S. Patents 4,079,095 and 4,079,096. The crystal structure of this class of zeolites is such as to provide access to and egress from the intracrystalline free space by virtue of the zeolites having pores the major dimension of which is greater than 3 but less than 6 Angstrom units. These zeolites are further characterized by pore windows of about a size such as would be provided by 8-membered rings of oxygen atoms. It will be understood, of course, that these rings are those formed by the regular disposition of the tetrahedra making up the anionic framework of the crystalline aluminosilicate, the oxygen atoms themselves being bonded to the silicon or aluminum atoms at the centers of the tetrahedra. The pores characterizing these zeolites may be substantially circular, such as in zeolite ZK-5 having uniform pores of about 3.9 Angstroms diameter or somewhat elliptical, such as in erionite having pores of approximately 3.6 by 5.2 Angstroms. It will be understood that, in any case, the small pore zeolites have a major pore dimension of less than 6 Angstroms. The pore size dimensions of these zeolites, as well as other feasible zeolites, are those specified in "Zeolite Frameworks" by W. M. Meier and D. H. Olson appearing in Advances in Chemistry Series, Vol. 101, pages 155—170 (1971).

In accordance with the invention, the conversion of the ether-rich effluent from the first stage to a highly olefinic hydrocarbon product is accomplished sequentially in a multi-stage adiabatic reactor system with interstage cooling. Cooling can be accomplished either directly or indirectly. The zeolite catalyst is provided in each of the stages as a fixed or moving bed. The quantity of catalyst in each bed is such that a uniform temperature rise occurs across each bed as the heat of reaction is released during the conversion of the feed into the highly olefinic product. The temperature rise across each bed and the quantity of catalyst in each bed is based on the desired overall conversion of the feed, the total adiabatic temperature increase for the methanol into olefin conversion and the sensitivity parameter for said conversion. By providing the catalyst in a sequential series of beds and providing interstage cooling to remove the heat of

reaction, stable operation is obtained and undesirable conversion to gasoline boiling-range hydrocarbons is substantially avoided.

The sensitivity parameter for a chemical reaction and a given catalyst is a constant and can be approximated by assuming Arrhenius dependence of reaction rate on temperature and that the reaction rate is approximately linear over the temperature range of interest. Therefore, the sensitivity parameter can be calculated from

$$\theta = \frac{R\ To^2}{E}$$

where

θ=sensitivity parameter
R=gas constant
To=initial temperature
E=activation energy

For the conversion of methanol to ethylene, θ is equal to about 18°C.

When an exothermic chemical reaction is carried out in an adiabatic reactor, the conversion may be obtained from the equation:

$$X_A = \frac{\Delta T\ Cp}{(-\Delta H)}$$

where

$X_A$=conversion
ΔT=adiabatic temperature rise
ΔH=heat of reaction
Cp=specific heat of feedstream in units of specific heat of the feedstream per mole of entering reactant

Solving for ΔT:

$$\Delta T = \frac{X_A\ (-\Delta H)}{Cp}$$

Therefore, the total adiabatic temperature rise for an exothermic reaction at a specific degree of conversion and a given feed composition can be calculated from the specific heat of the feed and the heat of reaction.

For an exothermic reaction to be controllable and not overly sensitive to perturbation in flow rate, catalyst state, feed composition or inlet temperature when carried out in an adiabatic bed of catalyst, the temperature rise in the bed must be limited to the sensitivity parameter, θ. Where the total adiabatic temperature rise for the reaction is greater than the sensitivity parameter, multiple catalyst beds must be employed to obtain the desired conversion under controllable conditions. The minimum number of beds required is equal to

$$\frac{\Delta T\ \text{total}}{\theta}$$

Where the ratio of the total adiabatic temperature rise to the sensitivity parameter is not an integer the next higher integer should be taken as the minimum number of beds required.

In accordance with the invention, the reaction mixture passing through the second stage reactor may flow through the individual catalyst beds in an axial or preferably in a radial flow configuration. A radial flow configuration results in a low pressure drop across each catalyst bed because of the high cross-sectional area and small bed thickness in the direction of flow. Either fixed bed or moving bed radial flow configurations may be employed.

The effluent from the second reaction zone is a gaseous stream containing ethylene, water, unconverted methanol and unconverted dimethyl ether. Some light paraffin and other hydrocarbons having from 3 to 10 carbon atoms will also be present in low concentrations. Passing the effluent into a condenser produces three phases—an aqueous phase containing most of the unreacted methanol, a liquid hydrocarbon phase containing $C_5$+hydrocarbons and a gaseous phase containing the ethylene produced, other light hydrocarbons and the unconverted dimethyl ether.

When methanol is contacted with the dehydration catalyst, an equilibrium mixture is obtained:

$$2CH_3OH \rightleftharpoons CH_3OCH_3 + H_2O \qquad (1)$$

The zeolite catalyst converts the methanol and the dimethyl ether into an olefin product:

$$2CH_3OH \rightarrow CH_2{=}CH_2 + 2H_2O \quad (2)$$

$$CH_3OCH_3 \rightarrow CH_2{=}CH_2 + H_2O \quad (3)$$

All of these reactions are exothermic so that any methanol converted in the second reaction zone undesirably produces additional heat in that zone. Methanol and dimethyl ether are only partially converted (<90%) into hydrocarbons in this process so as to achieve high ethylene selectivity. As the conversion of methanol and dimethyl ether is increased beyond 90%, increasing quantities are converted into gasoline boiling-range hydrocarbons, for example aromatic compounds. It will be appreciated that where a highly olefinic product is desired the conversion will be limited to yield the desired quantities of ethylene. Conversions in the range of about 30 to about 90% are usually employed.

Since the process must be operated at partial conversion to obtain the desired olefin product, the effluent from the second reaction zone contains significant quantities of unconverted methanol and dimethyl ether. It is therefore good engineering practice to recover unreacted feed from the product stream and to recycle it to the initial process step so as to improve the economics of the process. Such a practice has been employed in known processes in which a lower aliphatic alcohol is converted to a highly olefinic product; although unreacted alcohol and unreacted ether have been separately recovered and recycled in these processes, both recycle streams have, in all instances, been combined with fresh feed. According to a preferred aspect of the invention, however, significant advantages are achieved if the recovered alcohol is recycled to the fresh feed to the process, i.e., for processing in the first reaction zone which contains the dehydration catalyst, and the recovered ether is recycled to the equilbrium mixture obtained from the first reaction zone so that it may be processed in the second reaction zone which contains the zeolite catalyst. By segregating the ether recycle from the fresh feed and combining it with the alcohol-ether-water equilibrium mixture while the alcohol recycle is combined with the fresh alcohol feed, several processing advantages are obtained. As is evident from equation (1), recycling the alcohol and the ether results in an increase in the conversion of alcohol into ether and water in the dehydration reactor. In addition, by directing the recycle streams as described above there is a reduced heat load on the second reaction zone since more of the feed to this reaction zone enters as the ether which does not release as much heat when it is converted into the olefinic product as does the alcohol. The heats of reaction involved are:

| Equation | Reaction | $(\Delta H)_R$ at 370°C, Kcal/g mole |
|---|---|---|
| 1 | Methanol to dimethyl ether | −4.9 |
| 2 | Methanol to Ethylene | −2.8 |
| 3 | Dimethyl ether to Ethylene | −0.8 |

Following the conversion in the two reaction zones, the effluent from the second reaction zone is introduced into a condenser which results in the formation of three separate phases. This effectively separates substantially all of the unreacted methanol and the unreacted dimethyl ether into distinct phases. Two immiscible liquid phases, an aqueous phase and a hydrocarbon phase, are obtained together with a gaseous phase. The unreacted alcohol is found in the aqueous phase and the unreacted ether in the gaseous phase. Since the aqueous phase contains a significant quantity of water, the majority of which would be undesirable if added to the fresh feed, the water content of this phase is advantageously reduced before recycling the recovered alcohol admixed with some of this water. Such techniques as evaporation and steam stripping may be employed to achieve such water removal. Since the unconverted ether is present in the gaseous phase with ethylene and other hydrocarbon products from the process, the ether must be removed from this gaseous phase before it is recycled. This may be accomplished by passing the gaseous phase through a compressor and, once the pressure is increased, contacting the gaseous phase with an absorbing liquid which is effective to selectively absorb the dimethyl ether from the gaseous phase. Methanol or water may be conveniently employed as the absorbing liquid since either will selectively adsorb dimethyl ether from ethylene and the other product gases.

It is preferred that the dimethyl ether is separated from the absorbing liquid before the dimethyl ether is recycled so as to avoid any undesirable side effects the absorbing liquid may have on the methanol conversion. The separation may be achieved by passing the absorbing liquid containing the dimethyl ether, into a desorbing column where the separation may be achieved by simple pressure reduction.

The present invention will now be described in greater detail by way of example only with reference to the accompanying drawings, in which:

Figure 1 illustrates a scheme for carrying out the process;

Figure 2 illustrates a scheme similar to that of Figure 1 but using an alternative system for interstage cooling;

Figure 3 illustrates a scheme similar to that of Figure 1 but incorporating radial flow through the catalyst beds;

Figure 4 illustrates a scheme similar to that of Figure 2 but incorporating radial flow through the catalyst beds;

Figure 5 illustrates four different schemes for organizing radial flow through the catalyst beds;

Figure 6 illustrates a scheme similar to that of Figure 3 but incorporating a system for catalyst regeneration;

Figure 7 illustrates a scheme similar to that of Figure 1 but incorporating systems for the separate recycle of unconverted reactants; and

Figures 8, 9 and 10 are plots of temperature against catalyst bed length in the second stage of the process and illustrate the temperature characteristics of the process.

Referring first to Figure 1 of the drawings, a methanol feed containing up to about 50 weight percent water enters the process through line 2. It is mixed with an aqueous recycle stream which is rich in unreacted methanol from line 4 and a dimethyl ether (DME) rich recycle stream containing unreacted DME from line 6. The mixed stream is heated to reaction temperature 285—370°C in heat exchanger 8 and passed through line 10 into dehydration reactor 12 where it is converted into an essentially equilibrium mixture of methanol, DME, and water. The equilibrium mixture leaves the dehydration reactor through line 14 and its temperature is adjusted to 270—370°C in heat exchanger 16 prior to entering a multi-stage ZSM-5 fixed-bed adiabatic reactor 24 via line 18.

The mixture then contacts the first (stage 1) catalyst bed, hydrocarbons are formed and the temperature of the reactant/product mixture increases due to the exothermic nature of the conversion. The mixture leaving the first-stage catalyst bed is cooled by indirect heat exchange to a temperature substantially equal to that of the reactant mixture entering the stage 1 catalyst bed. The cooled reactant/product mixture then enters the stage 2 catalyst bed, additional conversion of reactants occurs liberating additional heat which again raises the temperature of the reactant/product mixture. This mixture leaving the stage 2 catalyst bed is also cooled by indirect heat exchange to a temperature approximately that existing at the entrance to the stage 1 catalyst bed. This conversion, heating, and cooling process continues until the last catalyst bed (stage N) is reached. The mixture leaving the penultimate catalyst bed (stage N-1) is cooled by indirect heat exchange and enters the catalyst bed of stage N. Upon leaving the stage N catalyst bed, the desired conversion level of methanol and DME has been achieved, and the heated product/reactant stream leaves the reactor through line 32, is cooled in heat exchanger 34 and passed via line 36 into product recovery section 38.

The cooled product/reactant mixture is present in three phases: (1) a liquid hydrocarbon phase, (2) an aqueous phase containing most of the unreacted methanol and some DME, and (3) a gaseous phase containing most of the light olefin hydrocarbon product and most of the unreacted DME.

The aqueous phase is subjected to several separation processes such as stream stripping, evaporation, and distillation to produce via line 4 an aqueous recycle stream which has a higher concentration of methanol than the raw aqueous phase produced by the dehydration operation. This recycle stream also will contain most of the DME initially present in the aqueous phase. It may not be necessary to employ all of the separation processes mentioned to achieve the desired composition of the aqueous recycle stream. This treatment of the aqueous phase recovers most of the water produced by conversion of methanol into hydrocarbons and the water initially present in the feed methanol in line 2 and these materials are removed from the system by line 40 to yield an aqueous product containing only small quantities of methanol and DME (<0.5 wt%).

The gaseous phase remaining after condensation is compressed and sent to an absorber to recover unreacted DME. While they may differ in efficiency, suitable absorbents are methanol, methanol feed, and water. The DME-rich solution is then stripped to obtain a DME-rich stream suitable for recycling back to either the dehydration reactor through lines 42 and 6 or to the multi-stage fixed-bed reactor through lines 42 and 44. The DME-lean stream from the absorber is then sent to an olefins recovery system similar to that employed in conventional olefin plants to recover ethylene, propylene, butenes, and a gasoline fraction through line 46.

As an alternative, the gaseous phase may be compressed and sent to distillation facilities to recover various hydrocarbons and DME for recycle as before.

The liquid hydrocarbon phase may be stabilized to recover light olefin products, and the stabilized liquid blended with gasoline boiling-range components recovered from the olefins recovery facility to make either finished gasoline or a gasoline blending stock.

The scheme shown in Figure 1 shows two different ways of handling the DME recycle stream. The scheme in which all the unreacted DME is fed to the inlet of the dehydration reactor has been described. In some instances, however, it may be desirable to feed the unreacted DME in line 42 directly to the multi-stage fixed-bed reactor by means of lines 44 and 18. The advantages of this latter operation are: (1) increased conversion of methanol to DME and water in the dehydration reactor, and (2) reduced heat load on the multi-stage reactor, since more of the feed enters as DME, which does not release as much heat when it is converted into hydrocarbons as does methanol. This reduced heat load increases the stability of the multi-stage reactor. Further details of such a recycle scheme are described below with reference to Figure 7 of the drawings.

Instead of the indirect interstage cooling shown in Figure 1, it is possible to achieve the desired cooling in between the stages by direct quenching with (1) liquid feed, (2) water, or (3) low pressure steam (for example process steam at 450 kPa). This alternative is shown in Figure 2, which illustrates only the multi-stage reactor of the system, the remainder of the system being identical to that of Figure 1. In Figure 2, the methanol/DME/water mixture from the dehydration stage enters reactor 124 through line 118 and the final product leaves through line 132. Direct cooling fluids, obtained from a variety of sources, flow through line 190 and are directed to each of the interstage direct cooling zones. A number of liquids obtained from the feed to the process may be employed for the quench cooling. The feed to dehydrator reactor 12, i.e., the combined streams from lines 2, 4 and 6, may be utilized. The other possible sources of liquid feed for direct cooling are the methanol feed from line 2, the aqueous phase recycle from line 4, the DME recycle from lines 6 or 42 or the condensate product from the dehydration reactor (a cooled stream from line 14). Other sources of direct cooling are water and low pressure (450 kPa) process steam. The principal difference between interstage direct cooling with water or steam and one of the process streams is that the process streams contain reactants which will be converted into olefins in the stages downstream of the direct cooling stage to which they are injected.

The following preferred operating conditions for a multi-stage adiabatic reactor as described above are based on data obtained from a small-scale reactor. It is well known that high operating pressures result in reduced yields of light olefins; therefore, the inlet pressure to the multi-stage reactor should be less than 520 kPa, preferably less than 380 kPa. It is not essential to house all stages in one vessel although this may be preferred for economic reasons. The interstage quenching involves a liquid injection system and a diffuser in the mixing zone between the stages. The diameter of the fixed catalyst bed of each stage is usually between 1.5 and 6 m (preferably between 2.5 and 4.5 m). Pressure drop and economic considerations indicate that the space velocity (WHSV, based on total feed) should be in the range of 0.2—2. The preferable operating temperature range is about 270 to about 370°C for a ZSM-5 type zeolite and about 315 to about 485°C for a small pore zeolite. Compensation for catalyst aging may be made by raising the inlet temperature to each stage. It is preferred to use a catalyst made with large crystal (at least about 1 micron) ZSM-5 zeolite in the multi-bed reactor, such as that described in U.S. Patents 4,025,571 and 4,148,835. The ZSM-5 type catalyst may be unsteamed or presteamed to reduce its hexane cracking activity (alpha value). Under the operating conditions described here the alpha value should exceed 20. The catalyst may also contain additional ingredients which improve ethylene selectivity, for example intracrystalline silica as described in U.S. Patents 4,060,568 and 4,100,219. Of course, other suitable catalysts may also be employed.

It is preferred to limit the overall conversion of methanol and DME to from 30 to about 90%. At higher conversions, significant quantities of methanol will be converted into aromatic compounds even at temperatures within the preferred range. To ensure partial conversion, it is essential that the reactor configuration and operating conditions do not result in an unstable or sensitive reactor operation (i.e., small perturbations in the operating conditions lead to excessive temperature rise and hence excessive conversion of desirable light olefins products). To operate this reactor system in a stable fashion, the temperature rise per bed should be restricted to about the value of the sensitivity parameter, which, in most instances, will be between about 10 and about 25°C, and often less than about 18°C. The minimum number of fixed beds is calculated from the total adiabatic temperature rise and the sensitivity parameter as explained above and is usually from 2 to 15, often from 4 to 10. Generally, the greater the number of beds, the less susceptible is the system to temperature perturbations in the feed to any of the fixed beds. However, economic considerations may dictate the use of fewer beds provided the expected temperature perturbations will not cause the effluent from any bed to exceed about 370°C for a ZSM-5 zeolite or about 485°C for a small pore zeolite.

The water content of the methanol feed to this process can vary between a nominal zero up to about 70 weight percent, preferably from about 20 to about 50 weight percent. Any "equivalent" methanol-water feed may also be employed. A methanol-water feed is said to be "equivalent" to a given methanol-water feed when the methanol-water feed in question plus any appropriate recycle stream recovered from the product of the process produces a feed to the zeolite catalyst reactor which has substantially the same composition as the equilibrium mixture obtained when the given methanol-water feed is contacted with a dehydration catalyst.

Referring now to Figure 3 of the drawings, the scheme shown is identical in all respects to that shown in Figure 1 described above except for the multi-bed reactor which in this case is designed for radial flow of reaction mixture through each of the individual catalyst beds. With this reactor configuration, the gaseous reaction mixture passes radially through each catalyst bed, the catalyst in each bed being retained within an annular region by perforated baskets.

The baskets are so designed that the reaction mixture flows from an annular space in the reactor through the outer periphery of the perforated baskets and radially through the body of catalyst. The catalyst baskets are sealed at the top and bottom, so that the reactant/product mixture cannot flow axially in the catalyst bed, and cannot bypass a central pipe for conveying the mixture to the next cooling stage. As the reaction mixture passes through the catalyst at least a part of it is converted into an olefinic product producing heat which causes an increase in the temperature of the mixture. The inner periphery of each basket is also perforated and defines the central pipe. The reaction mixture therefore flows through the

perforations of the inner periphery into the central pipe which conducts the heated mixture to the cooling stage.

The reaction mixture enters the first stage in the outer annulus 224 via lines 220 and 222, flows radially inwardly and contacts the first stage catalyst bed 226. Hydrocarbons are formed, and the temperature of the reactant/product mixture increases in the direction of flow due to the exothermic nature of the process. The mixture leaves the first stage 224 via central pipe 228, and is cooled by indirect heat exchange with coolant in coils 230 to a temperature substantially equal to the feed temperature to the first stage. The cooled reactant/product mixture then enters the second stage again via the outer annulus. The reactant/product mixture upon contacting the second stage catalyst bed undergoes additional conversion of reactants, liberating additional heat. The conversion, heating and cooling process continues in a manner similar to that described above until the last catalyst stage N is reached. Upon leaving stage N catalyst bed, the desired conversion level of methanol and DME has been achieved. The hot product/reactant stream leaves the reactor through line 232, is cooled and partially condensed in heat exchanger 234, and passes via line 36 into product recovery section 238 and is further processed as described above with reference to Figure 1. As with Figure 1 above, the procedure of recycling unconverted DME direct to the multi-stage reactor is described in detail below with reference to Figure 7.

Instead of indirect interstage cooling as shown in Figure 3, it is possible to achieve the desired cooling effect by direct quenching. Such direct cooling may be achieved in the same manner as that described above with reference to Figure 2 and is illustrated schematically for the radial flow configuration of Figure 3, in Figure 4.

For any given stage of the schemes shown in Figures 3 and 4, there are four possible radial flow orientations that can be employed; Figure 5 illustrates these orientations for an individual fixed catalyst bed. The radial flow may be from the outer periphery inwardly as in Figures 5a and 5b; from the center outwardly as in Figures 5c and 5d; in cocurrent flow as in Figures 5a and 5c or in countercurrent flow as in Figures 5b and 5d. The figure 5a pattern is depicted in Figures 3, 4 and 6 but any of the other three patterns can be employed where that particular configuration may prove advantageous. It will be appreciated that whereas the flow of Figure 5c is adaptable to a unit employing the Figure 5a flow pattern with minor piping modifications, the Figure 5b and 5d flow patterns would require extensive piping modifications. Even in a grass roots design, the Figure 5a and 5c flow patterns present no unusual design or piping problems but those of Figures 5b and 5d will require piping external to the reactor vessel to provide the desired radial flow pattern.

Since the zeolite catalyst employed in the process must be regenerated periodically, facilities may be provided for the catalyst to be moved through the series of catalyst beds by gravity and recovered from the last bed in the series for regeneration in a separate zone before being returned to the first bed in the series. Figure 6 shows a scheme combining radial flow and a moving bed multiple stage reactor. Since the scheme is identical in all respects to that described above with reference to Figure 3 except for the provisions of catalyst regeneration facilities, only the catalyst regeneration aspects of the scheme will be described. Referring now to Figure 6, zeolite catalyst, regenerated in regenerator 450, passes through line 452 together with any make-up catalyst supplied as required through line 454, and is introduced to the top of the multi-stage reactor 424 through hopper 456 which provides catalyst to the catalyst basket of the first stage. The catalyst is withdrawn and transferred by gravity flow to the next stage through conduits 458. The catalyst flows from stage to stage in general overall cocurrent relationship with the reaction mixture. Although the reaction mixture passes through an individual bed of catalyst in a radial flow pattern, the term "overall cocurrent relationship" and similar terminology is used to describe the flow whereby the fresh feed initially contacts fresh or freshly regenerated catalyst and passes through the several stages being gradually converted into the desired products by a catalyst which is slowly and continually being deactivated until the reaction mixture at the desired predetermined conversion and the deactivated catalyst both exit from the lower end of the multi-stage reactor. The catalyst flowing from the Nth stage is collected in hopper 460 and is transported through line 462 to catalyst regenerator 450. Alternatively, the catalyst from each stage is transferred directly to catalyst regenerator 450. In this situation replenishment of the catalyst in each stage is achieved independently. The catalyst circulation in the moving bed can be continuous or intermittent depending upon the rate of deactivation, which in turn depends upon the particular catalyst employed and the operating conditions. The moving bed multi-stage radial flow reactor possesses all the advantages of a fixed-bed radial flow reactor and also provides a means for continuous reactor operation. The fixed-bed multi-stage reactor, on the other hand, requires separate regeneration and operating cycles.

In another alternative, the feedstream can be introduced into the bottom of the multi-stage reactor so that the feed and the zeolite catalyst will flow in general overall countercurrent relationship to each other. This offers the usual advantages of countercurrent flow with the "richest" feedstock being contacted by the "lowest" activity catalyst and the "leanest" feedstock contacting the "highest" activity catalyst, thereby eliminating the problems of the excessively high reaction rates often encountered in the first bed of a cocurrent flow system.

Indirect interstage cooling is shown in Figure 6; it is possible, however, to use direct interstage cooling, as described above for the stationary catalyst bed in connection with Figure 4. In the scheme of Figure 6, the inlet temperature to stage 2 is usually higher than that of stage 1, with the inlet temperature increasing

with increasing stage number i.e. stage N being operated at the highest temperature, since the activity of the catalyst decreases with increasing stage numbers, stage 1 receiving the highest active catalyst and stage N having the least active catalyst. However, it is desirable to adjust the temperature of the interstage product/reactant mixture close to the inlet temperature of each stage as is done with cocurrent flow to ensure stable and insensitive reactor operation.

Based on data obtained in a small-scale isothermal reactor, the following preferred operating conditions for a multi-stage radial flow reactor system have been developed. Since low operating pressures are essential to achieve high yields of light olefins, the inlet pressure to the multi-stage reactor should be less than 380 kPa, and preferably less than 275 kPa. It is also not essential to house all zeolite catalyst stages in one vessel although this is usually preferred for economic reasons. The interstage quenching involves a liquid injection system and a diffuser with baffles in a mixing zone between the stages. The diameter of such a reactor system should be between about 1.2 and about 4.5 m (preferably about 1.8 to about 2.8 m). The thickness of the radial catalyst bed should be about 0.3 to about 1.2 m, preferably about 0.6 m. The diameter of the central pipe should be about 0.3 to about 1.0 m. Pressure drop and economic considerations indicate that the space velocity (WHSV, based on total feed) should be in the range of about 0.5 to about 5.0, preferably about 1.5 to about 3.5 $hr^{-1}$.

When the reaction mixture is passed through the beds of zeolite catalyst in a radial flow pattern, the pressure drop obtained is significantly lower than if an axial flow pattern were practiced with the same quantity of catalyst and the same overall conversion of reactants. In fact, the pressure drop in the radial flow reactor can be a factor of 3 and as much as an order of magnitude lower than with the conventional axial flow packed bed reactors, operated at equal WHSV. Conversely, for a given catalyst volume and allowable pressure drop the throughput through a radial flow reactor is significantly higher than with the conventional axial flow packed bed reactors. It is also usually more economical to construct a smaller diameter reactor of greater length, thus favoring radial flow over the large diameter fixed beds, with L/D ratios $<1/2$ (pancake reactors).

The preferred operating temperature range is about 270 to about 370°C for a ZSM-5 type zeolite and about 315 to about 485°C for a small pore zeolite. In this radial flow reactor configuration, compensation for catalyst aging may be made by varying the inlet temperature of the reactant/product stream to each stage.

As mentioned above, in the moving bed reactor scheme, activity of the catalyst decreases from top to bottom (i.e., from stage 1 to stage N), relative activity being dependent upon the catalyst circulation rate through the system. An optimum situation can be realized where catalyst circulation rate is very low and significant differences result in the activity of catalyst in the first and the Nth stages. In this situation relative quenching in the later stages can be reduced so that the feed temperature to each stage increases with increasing number of stages. In such configurations, the temperature will generally vary from 285°C in the first stage and could be as high as 355°C in the last stage.

In Figure 6, reactant and catalyst movement is cocurrent, i.e., fresh feed sees the most active catalyst. A desirable variation of the scheme, from reactor stability consideration, is countercurrent operation where the feed stream enters at the bottom of the reactor and meets the least active catalyst in stage N. The reactant/product stream is depleted in reactant concentration as it moves upwardly and meets more and more active catalyst.

The catalyst, conversion levels and feed compositions suitable for use in the radial flow reactor are generally identical to those described above for use in the axial flow reactor shown in Figures 1 and 2.

With typical operation conditions, a 50/50 (wt/wt) methanol-water feed requires six stages for 50% conversion. As the reaction proceeds along the reactor, concentration of reactant decreases and hence the amount of catalyst in each stage increases in the direction of reactant flow (temperature rise per bed is approximately constant). In the case of a fixed-bed radial flow reactor (as shown, for example in Figure 3) the relative proportions of catalyst contents of the six beds, with indirect interstage cooling, are 1.0: 1.10: 1.21: 1.32: 1.49: 1.78. As the radial thickness of the catalyst basket is constant, these ratios correspond to the ratios of the heights of the different stages. The practical limits on the minimum-maximum heights are dictated by the production capacity and maldistribution of the flow. Axial lengths in the range of about 0.6 to about 3 m, preferably about 0.9 to about 1.2 m, are suggested. The relative proportions of catalyst in the different stages of a moving-bed reactor configuration are determined by the deactivation characteristics of the catalyst and are specific for a given catalyst.

Referring now to Figure 7 of the drawings, a fresh methanol feed, which may contain up to about 50 weight percent water, enters through line 502 where it is mixed with an aqueous product recycle stream rich in unreacted methanol from line 504. This recycle stream is recovered in a subsequent step described below. The mixed stream passes through line 506, is heated to reaction temperature (285—370°C) in heat exchanger 508 and passes through line 510 into dehydration reactor 512 which contains a fixed bed 514 of gamma alumina. The mixture is converted to an essentially equilibrium mixture of methanol, dimethyl ether (DME) and water by the catalytic action of the alumina and leaves reactor 512 through line 516. A recycle stream of unreacted DME, recovered in an absorption-desorption operation described below, flows from line 518 and is mixed in line 20 with the equilibrium mixture from line 516. The resultant mixture passes through heat exchanger 522 where it is heated or cooled, as required, to the desired reaction temperature (270—370°C) for the second reaction zone. The mixture then flows through line 524 and into reactor 526 which contains ZSM-5 catalyst. Reactor 526 contains a plurality of catalyst beds with interstage

cooling of the reaction mixture; the beds may be fixed or moving; the reaction mixture may flow axially or radially through those beds; and interstage cooling may be achieved directly or indirectly, as described above.

The gaseous effluent from reactor 526, which comprises unconverted methanol, unconverted dimethyl ether, ethylene, other hydrocarbons and steam, passes through line 532 into condenser 534 where the methanol, the steam and the $C_5$+hydrocarbons are condensed. The condensed mixture and the uncondensed gases flow through line 536 into separator 538 where the mixture is resolved into three phases. The make-up of a typical effluent leaving reactor 526 and separating into three phases in separator 538 is as follows (figures in weight %):

| | |
|---|---|
| Methanol | 8.7 |
| Dimethyl Ether | 8.1 |
| Water | 70.0 |
| $CO$, $CO_2$, $H_2$ Total | <0.1 |
| Methane | 0.1 |
| Ethane | <0.1 |
| Ethylene | 3.3 |
| Propane | 0.6 |
| Propylene | 2.1 |
| Isobutane | 0.6 |
| n-Butane | 0.2 |
| Butenes | 1.0 |
| $C_5$+ | 5.1 |

The lowermost layer in separator 538 is an aqueous layer made up of the water produced by the conversion of methanol into hydrocarbons, any water contained in the feed, and unreacted methanol, plus a minor amount of dimethyl ether. The second layer, which appears between the aqueous layer and the gas phase, consists of hydrocarbons comprising $C_5$+hydrocarbons, i.e., higher molecular weight aliphatic and aromatic hydrocarbons. The uppermost layer is gaseous and consists of light hydrocarbons and most of the ethylene saturated with water vapor plus a substantial fraction of the unconverted dimethyl ether.

The first, aqueous layer is withdrawn and passes through line 540. Since an undesirable amount of water exists in this aqueous phase, a portion thereof is introduced, through line 542, into steam stripper 544. Steam introduced into stripper 544 removes the unreacted methanol and traces of DME from the portion of the aqueous phase cascading down through the stripper. The stripped products pass through line 546 and into line 504 where they are combined with the remaining portion of the aqueous phase and pass into line 502 for admixture with the fresh methanol feed, as described above. The water, stripped of the unreacted methanol and DME, is discharged from stripper 544 through line 548 and is discarded. It will be appreciated that the portion of the aqueous phase directed to the stripper can be varied so as to limit the total volume recycled through line 504 to that desired for effective operation of the process. As a first approximation, the quantity of the aqueous phase directed to the stripper should permit the rejection through line 548 of at least the quantity of water produced by the conversion of methanol and dimethyl ether into hydrocarbons. Variables in the process, such as the nature of the absorbing liquid employed to recover the DME, may dictate the stripping of more or less of the aqueous phase. This stripping operation can be adjusted as process and product requirements dictate.

The liquid hydrocarbon phase, containing $C_5$+hydrocarbons, is removed from separator 538 through line 550 and is sent to downstream units for recovery or further processing. For example, this stream can be employed as a fraction useful in gasoline blending.

The third phase in separator 538 is a gaseous phase which contains substantially all of the ethylene produced, together with light hydrocarbons, trace amounts of $CO$, $CO_2$, $H_2$, and a relatively large amount of dimethyl ether. This gaseous phase is withdrawn through line 552, compressed to about 1500 kPa by compressor 554 and is passed through line 556 to gas absorption column 558 containing a bed 560 of tower packing, for example Berl saddles, Raschig rings, or Pall rings. Water passing through line 562, is introduced into absorption column 558 where it passes countercurrent to the gas passing up the packed

column and effectively removes the dimethyl ether from the gas. The recovered dimethyl ether, dissolved in the water, passes from column 558 through line 564 and is introduced into desorber column 566 where, under reduced pressure, the dimethyl ether is separated from the water. The water passes from desorption column 566 and is recycled through line 562 for reuse in absorption column 558. Make-up water is added to the absorption system as required through line 568.

The separated dimethyl ether passes from desorption column 566 through line 518 and is recycled to line 520 where it is combined with the equilibrium mixture of methanol, dimethyl ether and water exiting from dehydration reactor 512 for conversion into an ethylene-rich product as described above.

The partially purified ethylene stream is withdrawn from absorption column 558 by means of line 570 and is passed to gas plant 572. Ethylene of the desired purity is recovered from the gas plant through line 574 and the heavier hydrocarbons, such as propylene and butenes, are withdrawn from the gas plant through line 576. The gas plant may also serve to remove CO, $CO_2$, $H_2$, $CH_4$ and $C_2H_6$.

The following Examples illustrate the invention.

Example I

The conversion of methanol into olefinic hydrocarbons is highly exothermic and unlike ammonia synthesis or methanol production has no thermodynamic equilibrium constraints. Higher temperatures, therefore, do not limit the conversion or temperature rise in a given catalyst bed. For the reaction to be controllable and not overly sensitive to perturbations in flow rate, catalyst state, feed composition and inlet temperature, the temperature rise in each bed must be limited to about the value of the sensitivity parameter, i.e., about 18°C.

Table I, below, summarizes the effect of this allowable ΔT/bed on reactor performance. The case considered is a stable 50% overall conversion of a 50 wt.% water, 50 wt.% methanol feed to a dehydration reactor, followed by 4, 6 and 8 stages of zeolite catalyst in a conversion reactor. Corresponding temperature rise per bed is 27, 18 and 13.6°C, respectively. Employing the equation given above, the total adiabatic temperature rise for the production of olefins from this methanol feed at 50% conversion is about 108°C. This means that if all of the catalyst were provided in a single bed the effluent temperature would be about 401°C for an initial feed inlet temperature of 293°C. A temperature rise such as this would be undesirable since it would result in unstable operation producing substantial quantities of high boiling products. Further, the gradual increase in feed inlet temperature which is required to compensate for catalyst aging further aggravates the unstable operation. Table I shows that with multiple beds and indirect interstage cooling, effluent temperatures of 320°C (4 stages), 311°C (6 stages) and 307°C (8 stages) will assure an olefinic product without conversion into aromatic compounds.

TABLE I

Sensitivity to temperature perturbations

Feed: 50:50 (w/w) MeOH: Water
Conversion: 50%
Inlet Temperature: 293°C
Coolant Temperature: 288°C
Indirect Interstage Cooling

| | 4 Stages | 6 Stages | 8 Stages |
|---|---|---|---|
| ΔT/Stages, °C | 27 | 18 | 13.6 |
| | Conversion | | |
| 5°C Perturbation | 61 | 53 | 51 |
| 7°C Perturbation | 75 | 59 | 53 |
| 10°C Perturbation | 100 | 65 | 55 |

For a given feed dilution (in this case 50/50) the more stages employed (with a corresponding smaller temperature rise per bed), the more stable is the reactor performance. This point is illustrated by imposing 5, 7 and 10°C perturbations on the inlet temperature of the first stage. The interstage cooling is indirect and in these indirect heat exchangers the coolant is maintained at 288°C. From the conversion obtained, as shown in Table I, it is seen that a six stage system, i.e., 18°C temperature rise/bed, is optimal since an eight stage system will require a significantly higher investment without a significantly greater stability than a six stage system. The sensitivity parameter for this reaction is approximately the same as the temperature rise obtained in each bed of the six stage system when processing a 50/50 methanol-water feed at an overall conversion of 50%. The temperature profile for this six stage system is shown in Figure 8.

Figure 9 shows graphically the temperature profiles for a four stage reactor system with the 7°C perturbation shown in Table I. The "x" temperature profiles are obtained under base case conditions (no perturbation). From this figure it can be seen that 7°C perturbation leads to an excessive temperature rise in the first stage (temperature in excess of 370°C) producing considerable quantities of undesirable products.

Example II

This example illustrates the effect of feed composition and overall conversion on the minimum number of beds of zeolite catalyst necessary to obtain stable operations while producing a highly olefinic product.

Table II below summarizes the minimum number of ZSM-5 zeolite catalyst stages required for various feed compositions at 50 and 80% conversion. In this table, the feed composition is expressed as weight percent methanol in water entering the dehydration reactor which provides the equilibrium mixture comprising methanol, DME and water entering the multi-bed zeolite catalyst system.

TABLE II
Effect of feed composition and conversion on number of stages

| Feed composition, wt.% methanol in water | Minimum No. of beds at 50% conversion | Minimum No. of beds at 80% conversion |
|---|---|---|
| 16 | 2 | 4 |
| 50 | 6 | 10 |
| 84 | 8 | 15 |
| 100 | 9 | 17 |

A typical case of a 50/50 (wt/wt) methanol water feed at 50% conversion requires six stages. As the reaction proceeds along the reactor, the concentration of reactant decreases and hence the amount of catalyst in each stage increases in the direction of reactant flow (temperature rise per bed is constant). For the above example the relative proportion of six beds with indirect interstage cooling would be 1.0: 1.10: 1.21: 1.32: 1.44: 1.78. A typical temperature profile for this six stage reactor is shown in Figure 8. Note the uniform temperature rise per bed, the interstage cooling which returns the reaction mixture to the inlet temperature to the first stage and the increase in catalyst bed size as the reaction mixture passes sequentially through the six stages. Similarly, a ten stage system to achieve 80% conversion would need 10 beds in the following proportions: 1.0: 1.09: 1.20: 1.33: 1.5: 1.72: 2.00: 2.41: 3.00: 3.98.

As mentioned above, several variations in interstage cooling are possible. Table III below summarizes the relative amounts of quench desirable at various stages, for a six stage system. For water and feed quench cooling, the increase in packed-bed thickness would be essentially the same as that detailed above. For steam quenching the thickness would increase in a higher proportion.

TABLE III
Quenching of a six stage reactor with various quenching agents

| | Quench/stage, % of feed to first stage quenching agent | | |
|---|---|---|---|
| Inlet to stage | Feed equivalent to 50/50 (W/W) methanol/water | Water | 450 kPa sat'd steam |
| 2 | 2.2 | 1.5 | 12.0 |
| 3 | 2.3 | 1.5 | 13.4 |
| 4 | 2.4 | 1.6 | 15.1 |
| 5 | 2.5 | 1.7 | 16.9 |
| 6 | 2.6 | 1.8 | 18.9 |

Example III

The effect of a higher water content feed is shown in this example which is similar to that of the four stage system of Example I in all aspects except feed composition.

Figure 10 presents the effect of water dilution and temperature perturbations with a 67/33 (w/w) $H_2O$/MeOH feed to the dehydration reactor. The overall conversion is 50% with the system under stable operating conditions. Again the temperature rise per bed is about 18°C. The temperature profiles for this system are shown in Figure 10. The "x" profiles are for the base case where the temperature rise across each of the four beds is the same. The temperature profile obtained for an 10°C perturbation to the inlet temperature to the first stage is also shown on this figure. It should be noted that the starting temperature for this four stage system is lower than the six stage system shown in Figure 8.

Figure 10 shows that although a 10°C perturbation causes a significantly higher effluent temperature in the first stage as compared to the base case, it is much lower than the first stage effluent temperature obtained with a 7°C perturbation in the four stage system of Example I. In fact, with the higher water content feed, the 10°C perturbation does not cause the temperature of the first stage effluent to exceed the 370°C limitation. Comparing Figure 9 with Figure 10 shows that higher water content methanol feedstocks can handle larger perturbations to the inlet temperature to the first bed of a sequential multi-bed system while still avoiding the high temperatures which promote undesirable side reactions.

Example IV

The pressure drops of a reacting fluid passing through packed beds may be calculated using Ergun's equation.

$$\frac{dp}{dz} = \frac{G^2}{D_p g_c \rho} \cdot \frac{1-\varepsilon}{\varepsilon^3} \left[1.75 + \frac{150\,(1-\varepsilon)\mu}{\psi D_p G}\right]$$

Assuming ideal gas law behavior for density change due to declining pressure, integration of the above provides:

$$P = P_o \left[ 1 - \sqrt{1 - \frac{2RTG^2\,(1-\varepsilon)}{\psi Po^2 \varepsilon^3 g_c M D_p} \left[ \frac{150\,(1-\varepsilon)\mu}{\psi DpG} + 1.75 \right] Z} \right]$$

in which

G=mass flux
$\psi$=shape factor
$D_p$=catalyst particle diameter
$\rho$=gas density
$g_c$=constant
$\varepsilon$=void fraction
$\mu$=viscosity
Po=inlet pressure
M=molecular weight
T=average temperature of the gas
R=gas constant
Z=distance in the direction of flow.

The equation may be used to compare the pressure drop in an axial flow reactor with a radial flow reactor.

The feed to both reactors is the same methanol-water feed and the following operating conditions are employed:

WHSV=3 $hr^{-1}$ (total)
Inlet temperature=293°C
void fraction=0.38.

Using the above equation, the pressure drop for a fixed bed reactor having a 0.9 m packed height is 2.76 kPa while for a radial flow reactor of equivalent capacity and a 0.3 m thickness the pressure drop is 0.21 kPa.

It is clear from the above that conducting the conversion of methanol into olefins in radial flow reactors can reduce the pressure drop by as much as an order of magnitude from that obtained in axial flow reactors.

Example V

The reaction,

$$2CH_3OH \rightleftharpoons CH_3OCH_3 + H_2O,$$

is an equilibrium limited reaction. From Le Chatelier's Principle, addition of dimethyl ether to methyl alcohol will reduce the conversion. This effect is shown by considering two different feedstreams being introduced into the dehydration reactor at a temperature of 315°C and a pressure of 310 kPa.

Case I—No DME recycle to dehydration reactor

| Feed | Moles in | Moles out |
|---|---|---|
| MeOH—50g. | 1.56 | 0.45 |
| DME—0g. | 0.0 | 0.56 |
| $H_2O$—50g. | 2.78 | 3.33 |

Case II—DME recycle to dehydration reactor

| Feed | Moles in | Moles out |
|---|---|---|
| MeOH—25g. | 0.78 | 0.47 |
| DME—25g. | 0.54 | 0.70 |
| $H_2O$—50g. | 2.78 | 2.94 |

Conversion of MeOH= $1-\frac{C_{in}}{C_{out}}$

Case I= $1-\frac{0.45}{1.56}=71.2\%$

Case II= $1-\frac{0.47}{0.78}=39.7\%$

This example shows that the addition of dimethyl ether to the feedstream entering the dehydration reactor reduces the methyl alcohol conversion effected in that reactor.

**Claims**

1. A process for converting methanol into an olefinic hydrocarbon product which comprises the steps of

contacting a feed comprising methanol at an elevated temperature with a dehydration catalyst to obtain an ether-rich product, and

contacting the ether-rich product at an elevated temperature with a catalyst comprising a crystalline aluminosilicate zeolite having either (i) a pore size greater than 5 Angstroms, a silica to alumina mole ratio of at least 12 and a constraint index from 1 to 12 or (ii) pores the major dimension of which is less than 6 Angstroms and pore windows of a size provided by 8-membered rings of oxygen atoms, to achieve a predetermined degree of conversion into olefinic hydrocarbon products, by passing the ether-rich product through a plurality of alternate beds of zeolite catalyst and cooling zones, the reduction in temperature of the reaction mixture by passage through each cooling zone being substantially equal to the increase in temperature in the immediately preceding catalyst bed, the increase in temperature of the reaction mixture by passage through each catalyst bed being no greater than the sensitivity parameter for the conversion of methanol into ethylene and the number of catalyst beds being at least equal to the ratio of the total adiabatic temperature increase at the predetermined conversion to the sensitivity parameter.

2. A process according to claim 1, wherein the reaction mixture passes axially through each zeolite catalyst bed.

3. A process according to claim 1, wherein the reaction mixture passes radially through each zeolite catalyst bed.

4. A process according to any one of claims 1 to 3, wherein each zeolite catalyst bed is a fixed bed.

5. A process according to any one of claims 1 to 3, wherein each zeolite catalyst bed is a moving bed.

6. A process according to claim 5, wherein the flow of reaction mixture is cocurrent to the flow of catalyst.

7. A process according to claim 5, wherein the flow of reaction mixture is countercurrent to the flow of catalyst.

8. A process according to any one of claims 1 to 7, wherein the olefinic hydrocarbon product is separate into its components, unreacted methanol is recycled to the dehydration catalyst and unreacted ether is recycled to the zeolite catalyst beds.

**Patentansprüche**

1. Verfahren zur Umwandlung von Methanol in ein olefinisches Kohlenwasserstoffprodukt, das die Schritte umfaßt:

Kontaktieren eines Einsatzprodukts, das Methanol enthält, bei einer erhöhten Temperatur mit einem Wasserabspaltungs-Katalysator unter Gewinnung eines etherreichen Produkts und

Kontaktieren des etherreichen Produkts bei einer erhöhten Temperatur mit einem Katalysator, der einen kristallinen Alumosilicat-Zeolithen mit entweder (i) einer Porengröße von mehr als 5Å, einem Siliciumoxid/Aluminiumoxid-Molverhältnis von wenigstens 12 und einem Grenzwertindex (constraint index) von 1 bis 12 oder (ii) Poren, deren Hauptabmessung weniger als 6Å beträgt und mit Porenfenstern einer Größe, die durch achtgliedrige Ringe aus Sauerstoffatomen geschaffen wird, umfaßt, um einen vorgegebenen Umwandlungsgrad in olefinische Kohlenwasserstoffprodukte zu erreichen, indem man das etherreiche Produkt durch eine Vielzahl von alternierenden Betten aus Zeolith-Katalysator und Kühlzonen leitet, wobei die Verminderung der Temperatur der Reaktionsmischung während des Durchströmens durch jede Kühlzone im wesentlichen gleich ist der Temperatursteigerung in dem unmittelbar vorausgehenden Katalysatorbett, wobei die Temperatursteigerung der Reaktionsmischung durch Durchleiten durch jedes Katalysatorbett nicht größer ist als der Empfindlichkeitsparameter für die Umwandlung von Methanol in Ethylen und wobei die Anzahl der Katalysatorbetten wenigstens gleich ist dem Verhältnis der gesamten adiabatischen Temperatursteigerung bei der vorgegebenen Umwandlung zu dem Empfindlichkeitsparameter.

2. Verfahren nach Anspruch 1, bei dem die Reaktionsmischung jedes Zeolith-Katalysator-Bett axial durchströmt.

3. Verfahren nach Anspruch 1, bei dem die Reaktionsmischung jedes Zeolith-Katalysator-Bett radial durchströmt.

4. Verfahren nach irgendeinem der Ansprüche 1 bis 3, bei dem jedes Zeolith-Katalysator-Bett ein Festbett ist.

5. Verfahren nach irgendeinem der Ansprüche 1 bis 3, bei dem jedes Zeolith-Katalysator-Bett ein Bewegtbett ist.

6. Verfahren nach Anspruch 5, bei dem der Strom der Reaktionsmischung dem Strom des Katalysators gleichgerichtet ist.

7. Verfahren nach Anspruch 5, bei dem der Strom der Reaktionsmischung dem Strom des Katalysators entgegengerichtet ist.

8. Verfahren nach irgendeinem der Ansprüche 1 bis 7, bei dem das olefinische Kohlenwasserstoffprodukt in seine Bestandteile aufgetrennt wird, unumgesetztes Methanol zu dem Wasserabspaltungs-Katalysator zurückgeführt wird und unumgesetzter Ether zu den Zeolith-Katalysator-Betten zurückgeführt wird.

**Revendications**

1. Procédé pour convertir du méthanol en un produit hydrocarboné oléfinique, qui comprend les étapes consistant à:

mettre une alimentation, comprenant du méthanol, en contact à une température élevée avec un catalyseur de déshydratation pour obtenir un produit riche en éther, et

mettre le produit riche en éther, à une température élevée, en contact avec un catalyseur comprenant un aluminosilicate zéolitique cristallin ayant (i) une teille des pores supérieure à 5 Angströms, un rapport molaire de la silice à l'alumine au moins égal à 12 et un indice de contrainte de 1 à 12, ou (ii) des pores dont la plus grande dimension est inférieure à 6 Angströms et des fenêtres de pores d'une taille fournie par des cycles octogonaux d'atomes d'oxygène, pour réaliser un degré prédéterminé de conversion en des produits hydrocarbonés oléfiniques, en faisant passer le produit riche en éther par plusieurs lits de catalyseur zéolitique alternant avec des zones de refroidissement, la diminution de température du mélange réactionnel par son passage à travers chaque zone de refroidissement étant sensiblement égale à l'élévation de température dans le lit de catalyseur immédiatement précédent, l'élévation de température du mélange réactionnel par son passage dans chaque lit de catalyseur n'étant pas plus grande que le paramètre de sensibilité pour la conversion du méthanol en éthylène et le nombre de lits de catalyseur étant au moins égal au rapport entre l'augmentation de température adiabatique totale du taux prédéterminé de conversion et le paramètre de sensibilité.

2. Procédé selon la revendication 1, dans lequel le mélange réactionnel traverse axialement chaque lit de catalyseur zéolitique.

3. Procédé selon la revendication 1, dans lequel le mélange réactionnel traverse radialement chaque lit de catalyseur zéolitique.

4. Procédé selon l'une quelconque des revendications 1 à 3, dans lequel chaque lit de catalyseur zéolitique est un lit fixe.

5. Procédé selon l'une quelconque des revendications 1 à 3, dans lequel chaque lit de catalyseur zéolitique est un lit mobile.

6. Procédé selon la revendication 5, dans lequel l'écoulement du mélange réactionnel est cocourant avec l'écoulement du catalyseur.

7. Procédé selon la revendication 5, dans lequel l'écoulement du mélange réactionnel est à contre-courant de l'écoulement du catalyseur.

8. Procédé selon l'une quelconque des revendications 1 à 7, dans lequel le produit hydrocarboné oléfinique est séparé en ses constituants, le méthanol inaltéré est recyclé dans le catalyseur de déshydratation et l'éther inaltéré est recyclé vers les lits de catalyseur zéolitique.

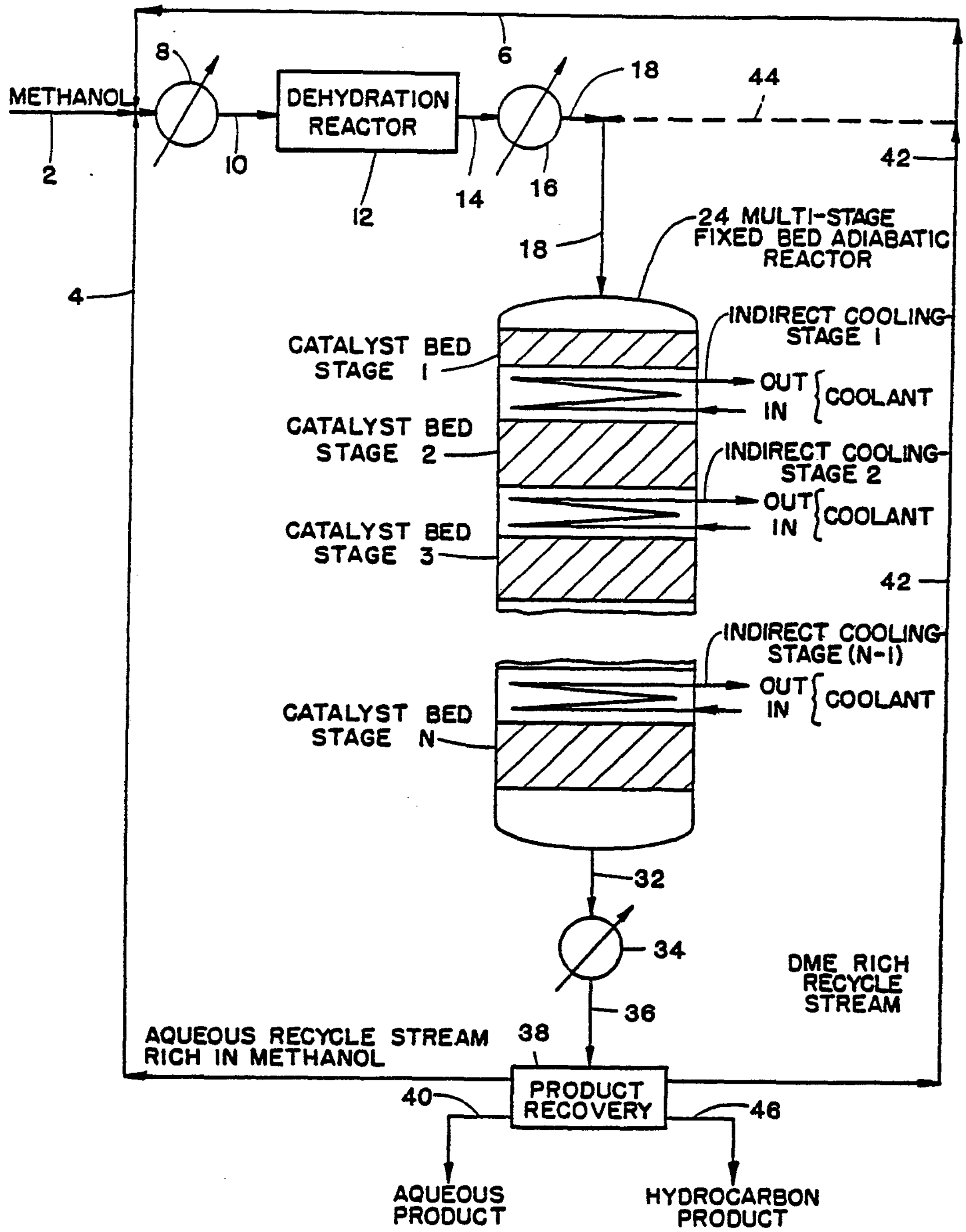
METHANOL
2
8
10
DEHYDRATION REACTOR
12
6
14
16
18
44
42
4
24 MULTI-STAGE FIXED BED ADIABATIC REACTOR
18
INDIRECT COOLING STAGE 1
CATALYST BED STAGE 1
OUT
IN
COOLANT
CATALYST BED STAGE 2
INDIRECT COOLING STAGE 2
OUT
IN
COOLANT
CATALYST BED STAGE 3
42
INDIRECT COOLING STAGE (N-1)
OUT
IN
COOLANT
CATALYST BED STAGE N
32
34
36
DME RICH RECYCLE STREAM
AQUEOUS RECYCLE STREAM RICH IN METHANOL
38
PRODUCT RECOVERY
40
46
AQUEOUS PRODUCT
HYDROCARBON PRODUCT

FIG. 1

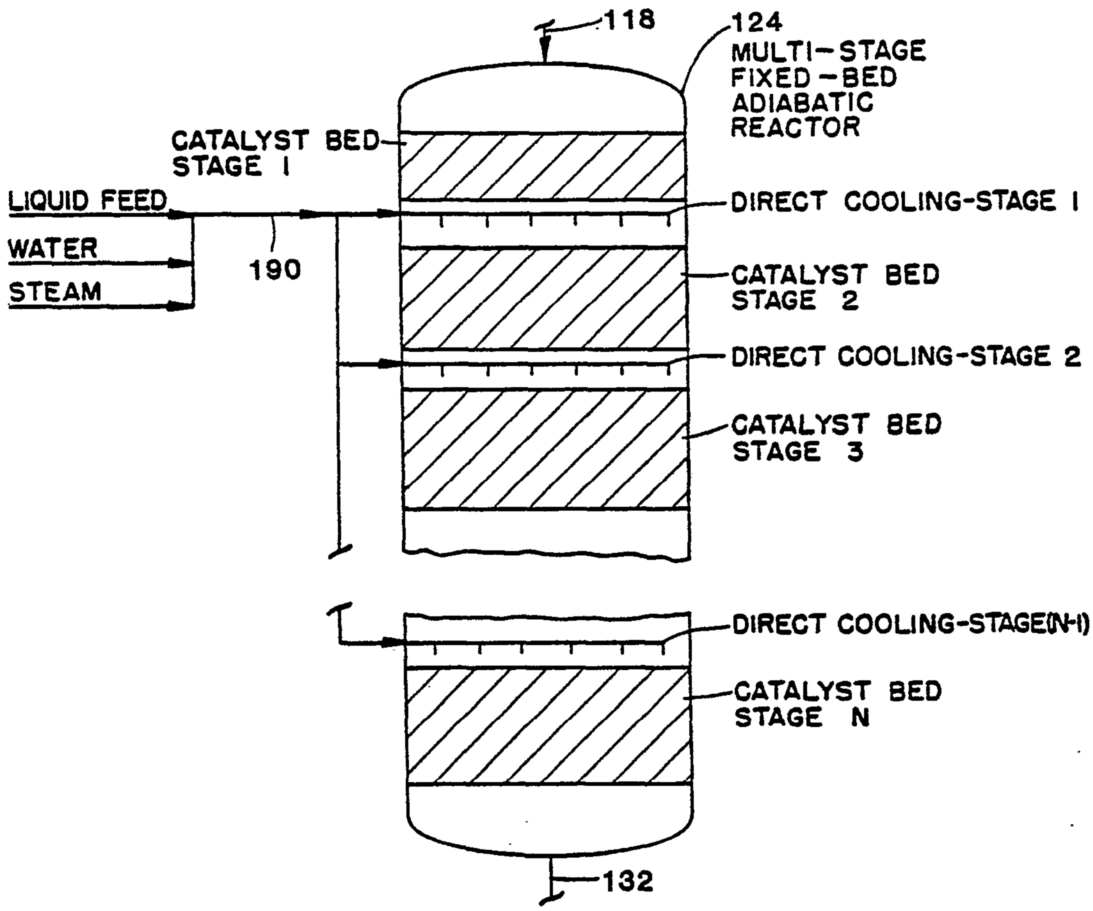
118
124
MULTI-STAGE
FIXED-BED
ADIABATIC
REACTOR
CATALYST BED
STAGE 1
LIQUID FEED
WATER
STEAM
190
DIRECT COOLING-STAGE 1
CATALYST BED
STAGE 2
DIRECT COOLING-STAGE 2
CATALYST BED
STAGE 3
DIRECT COOLING-STAGE(N-1)
CATALYST BED
STAGE N
132

FIG. 2

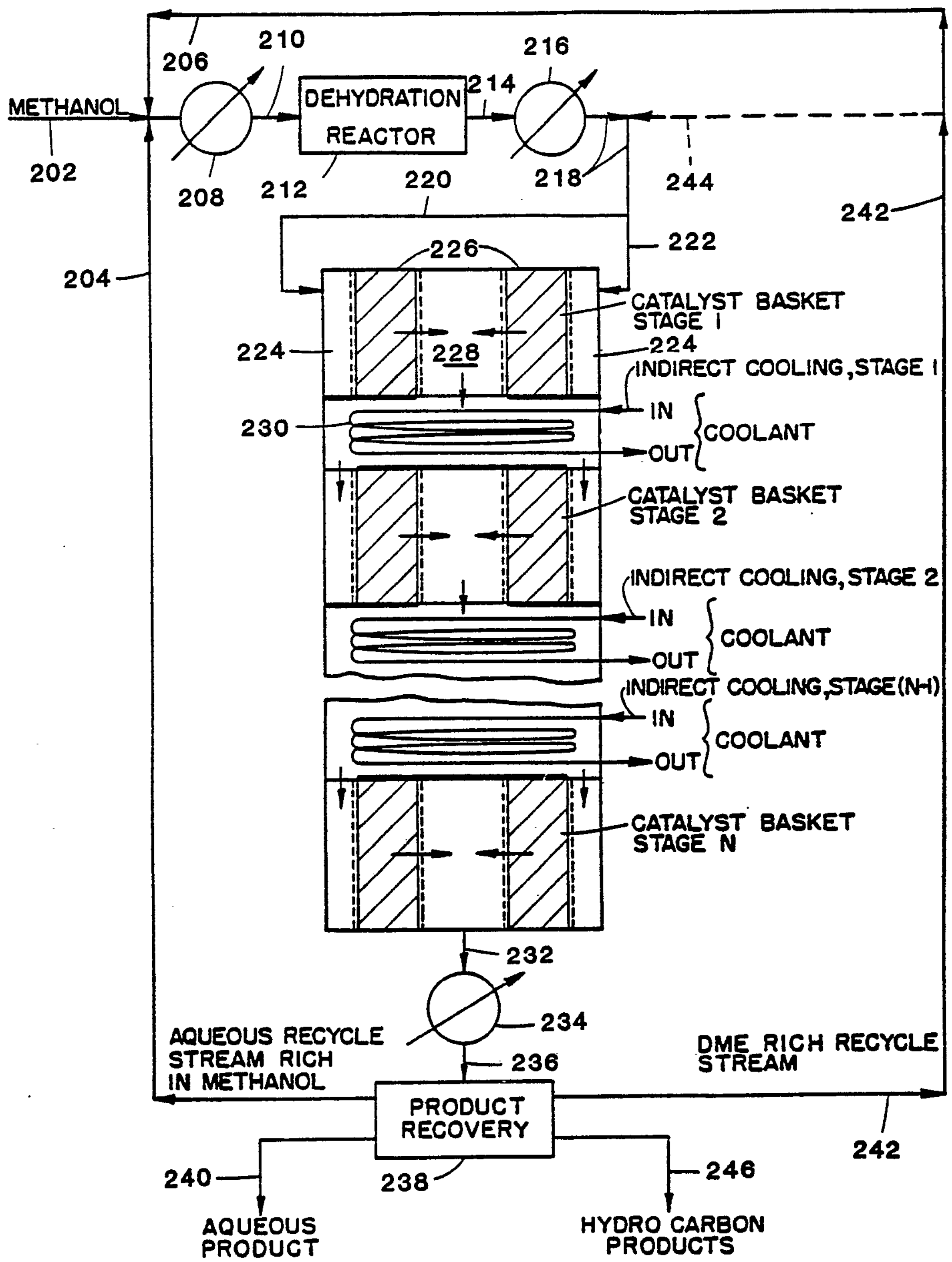
METHANOL
202
204
206
208
210
DEHYDRATION REACTOR
212
214
216
218
220
222
244
242
226
224
228
CATALYST BASKET STAGE I
INDIRECT COOLING, STAGE I
230
IN
OUT
COOLANT
CATALYST BASKET STAGE 2
INDIRECT COOLING, STAGE 2
INDIRECT COOLING, STAGE (N-I)
CATALYST BASKET STAGE N
232
234
236
AQUEOUS RECYCLE STREAM RICH IN METHANOL
DME RICH RECYCLE STREAM
PRODUCT RECOVERY
238
240
246
AQUEOUS PRODUCT
HYDRO CARBON PRODUCTS

FIG. 3

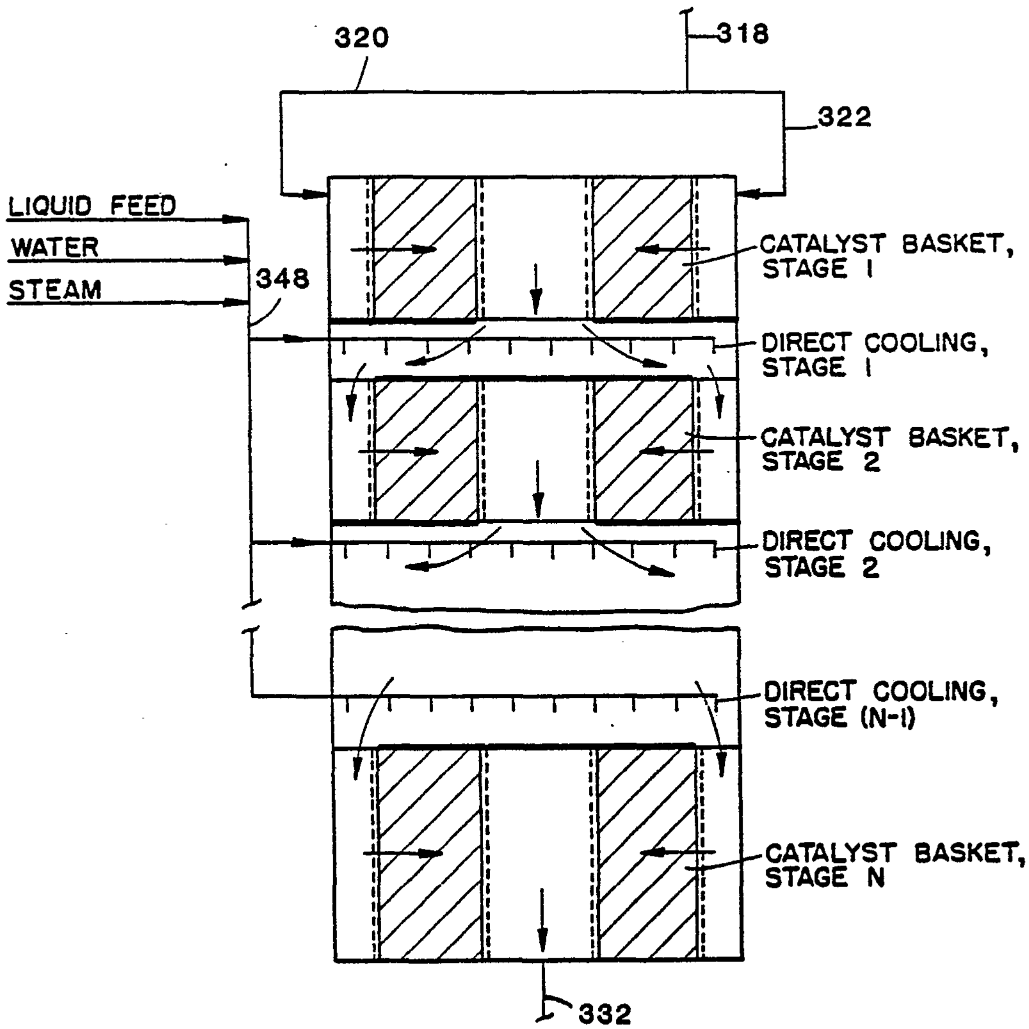
320
318
322
LIQUID FEED
WATER
STEAM
348
CATALYST BASKET, STAGE I
DIRECT COOLING, STAGE I
CATALYST BASKET, STAGE 2
DIRECT COOLING, STAGE 2
DIRECT COOLING, STAGE (N-I)
CATALYST BASKET, STAGE N
332

FIG. 4

A)

B)

C)

D)

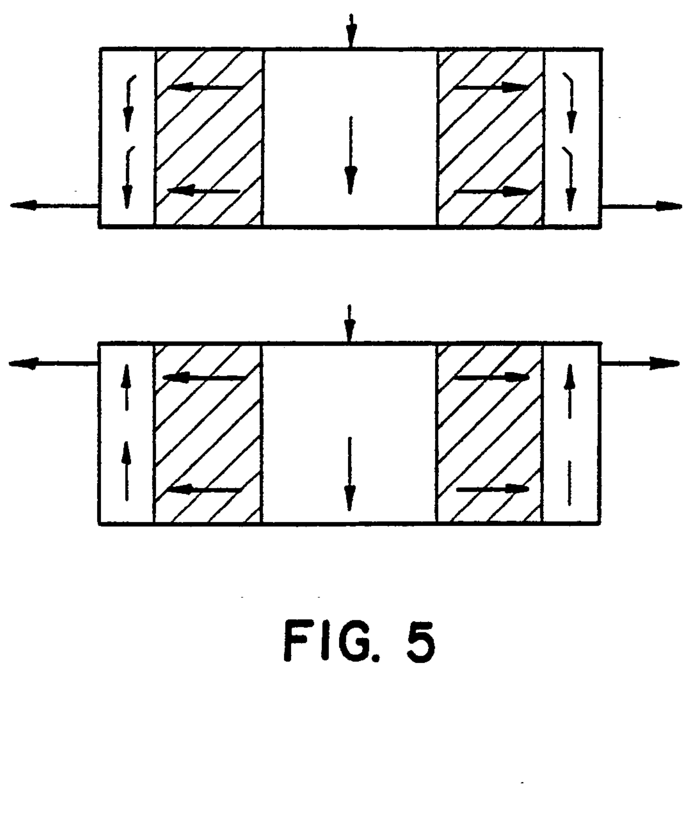

FIG. 5

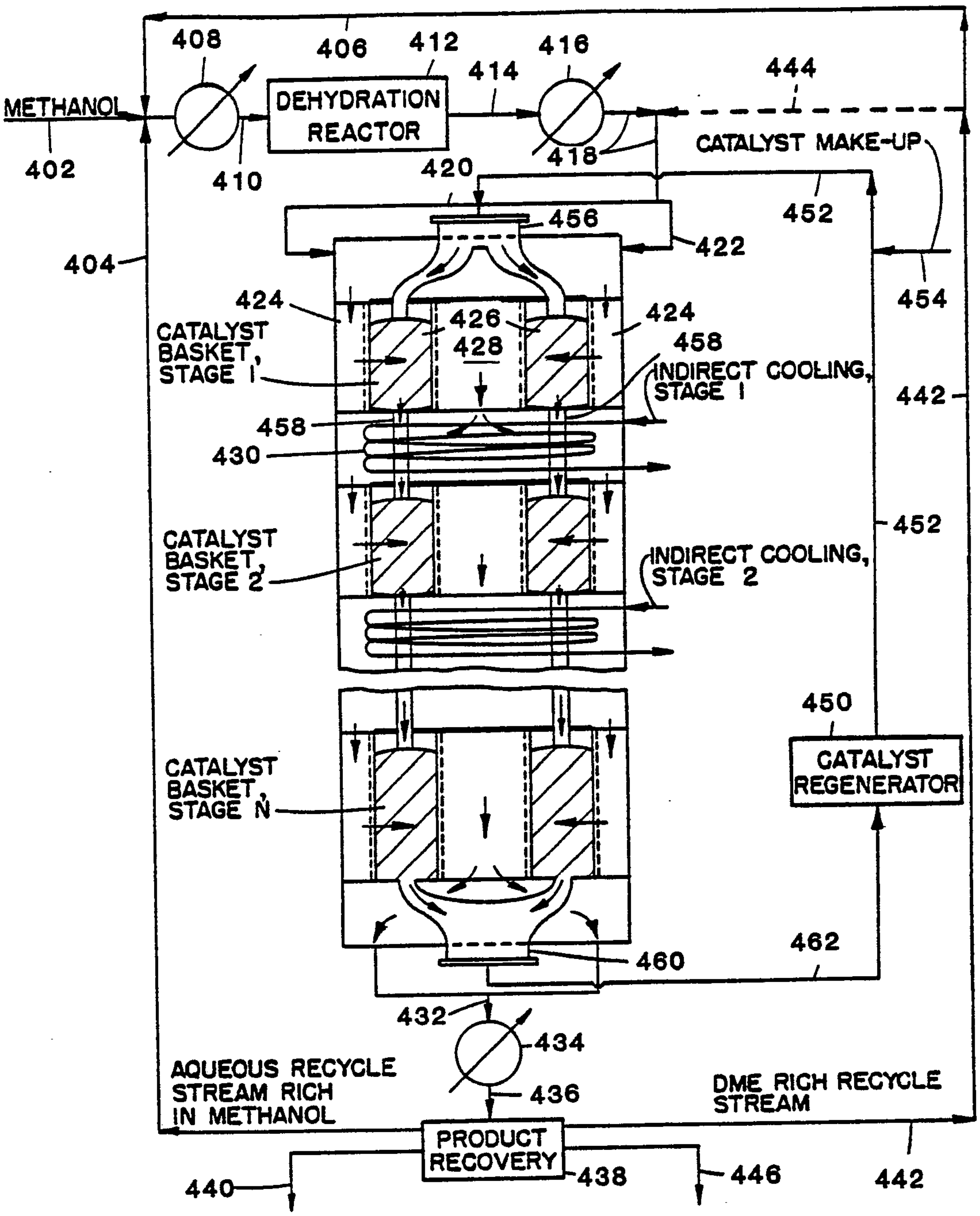

METHANOL
402
404
408
406
410
412
DEHYDRATION REACTOR
414
416
418
420
444
CATALYST MAKE-UP
452
454
456
422
424
426
428
458
CATALYST BASKET, STAGE 1
INDIRECT COOLING, STAGE 1
430
CATALYST BASKET, STAGE 2
INDIRECT COOLING, STAGE 2
442
CATALYST BASKET, STAGE N
450
CATALYST REGENERATOR
460
462
432
434
436
AQUEOUS RECYCLE STREAM RICH IN METHANOL
DME RICH RECYCLE STREAM
PRODUCT RECOVERY
438
440
446


FIG. 6

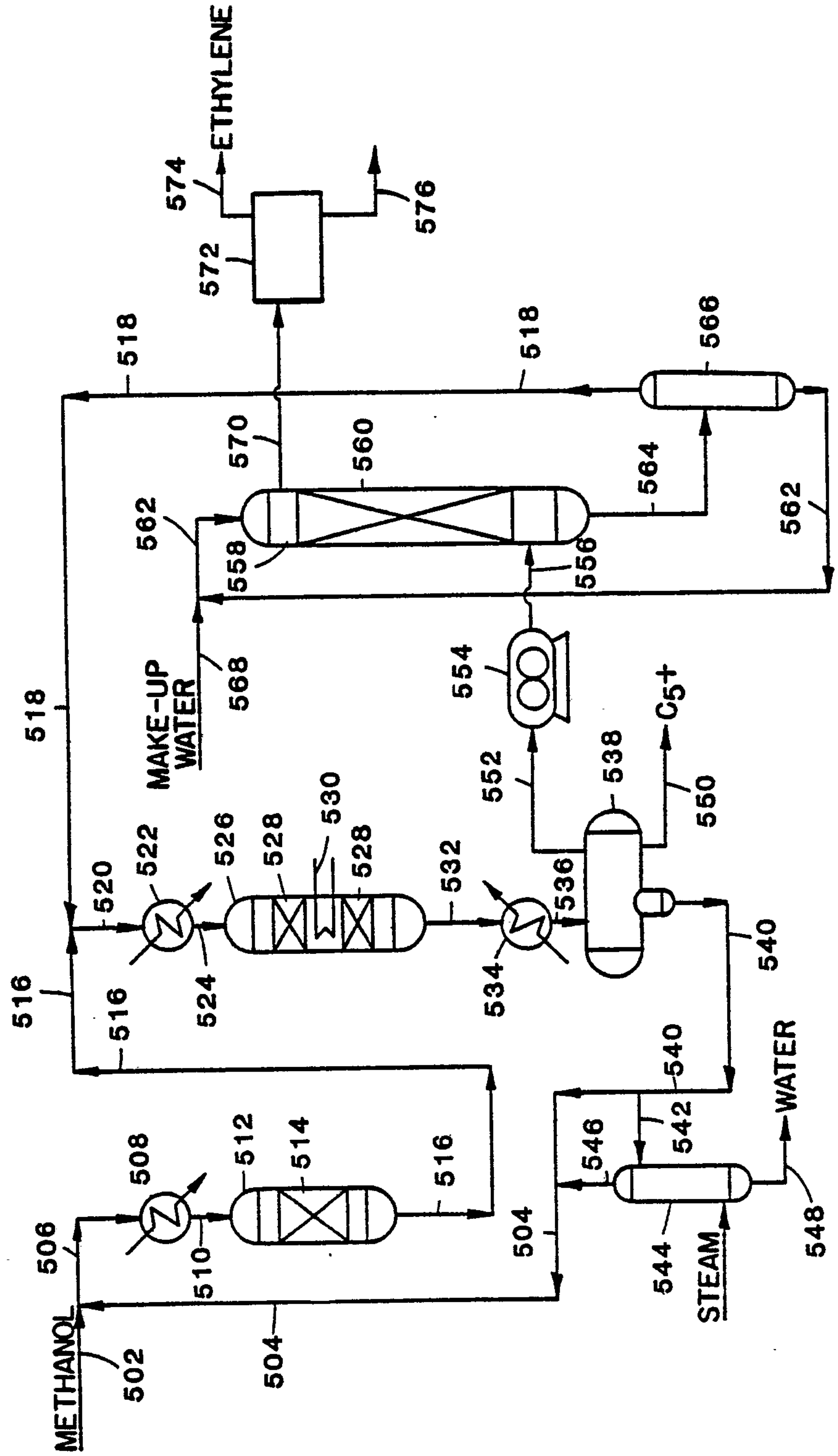

METHANOL
502
504
506
508
510
512
514
516
518
520
522
524
526
528
530
532
534
536
538
540
542
544
546
548
550
552
554
556
558
560
562
564
566
568
570
572
574
576
MAKE-UP WATER
ETHYLENE
STEAM
WATER
$C_5+$


FIG. 7

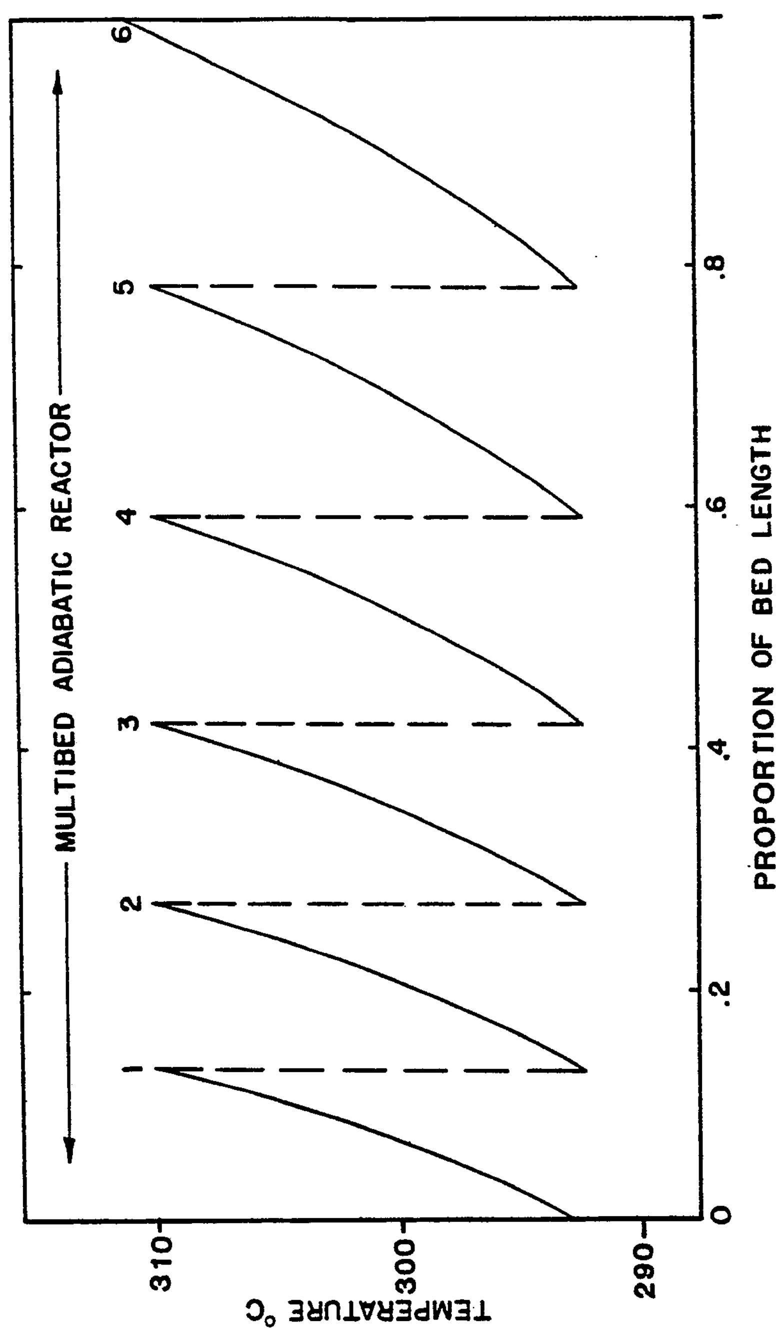
MULTIBED ADIABATIC REACTOR
TEMPERATURE °C
310
300
290
PROPORTION OF BED LENGTH
0
.2
.4
.6
.8
1
2
3
4
5
6

FIG. 8

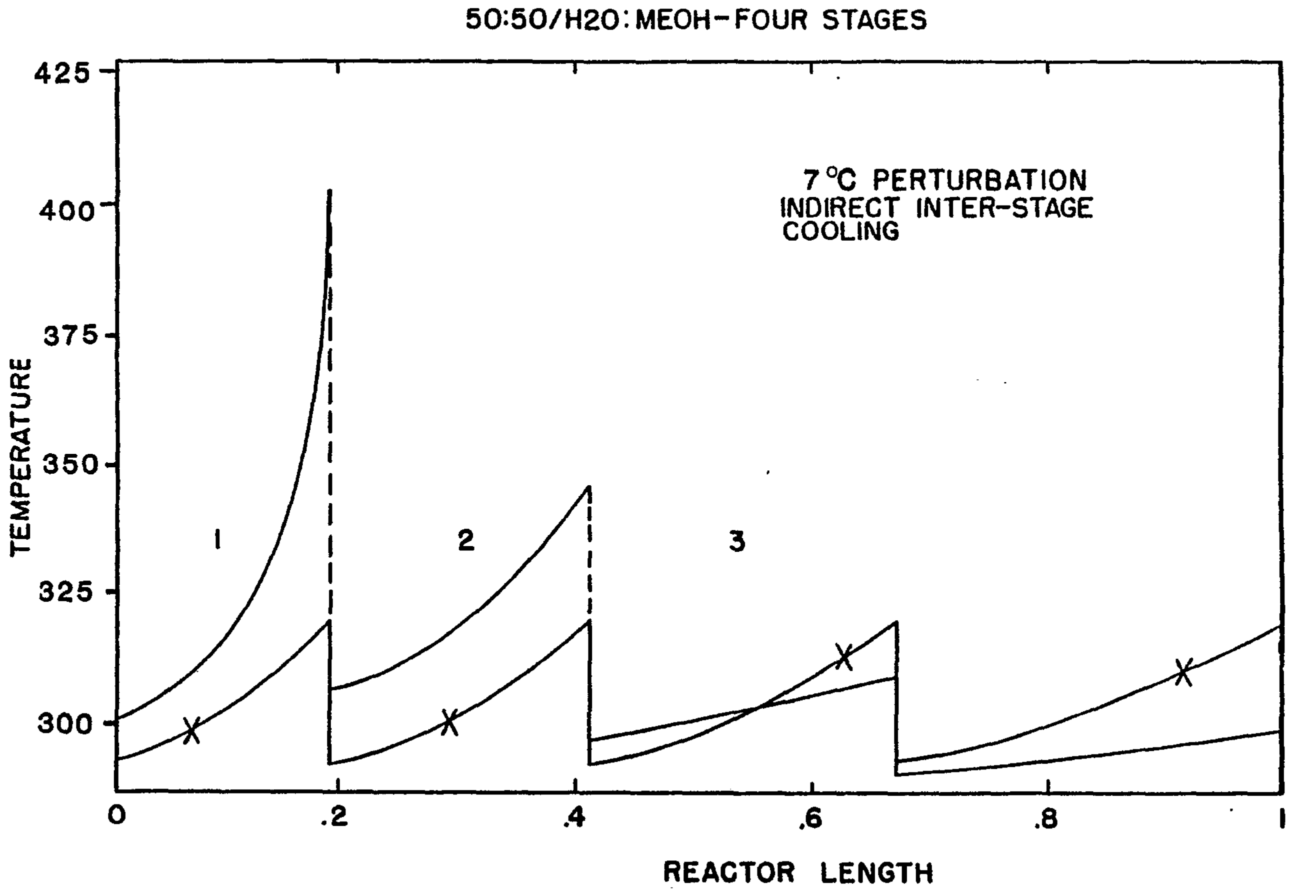

50:50/H2O:MEOH-FOUR STAGES
425
400
375
350
325
300
TEMPERATURE
7 °C PERTURBATION
INDIRECT INTER-STAGE
COOLING
1
2
3
0
.2
.4
.6
.8
1
REACTOR LENGTH


FIG. 9

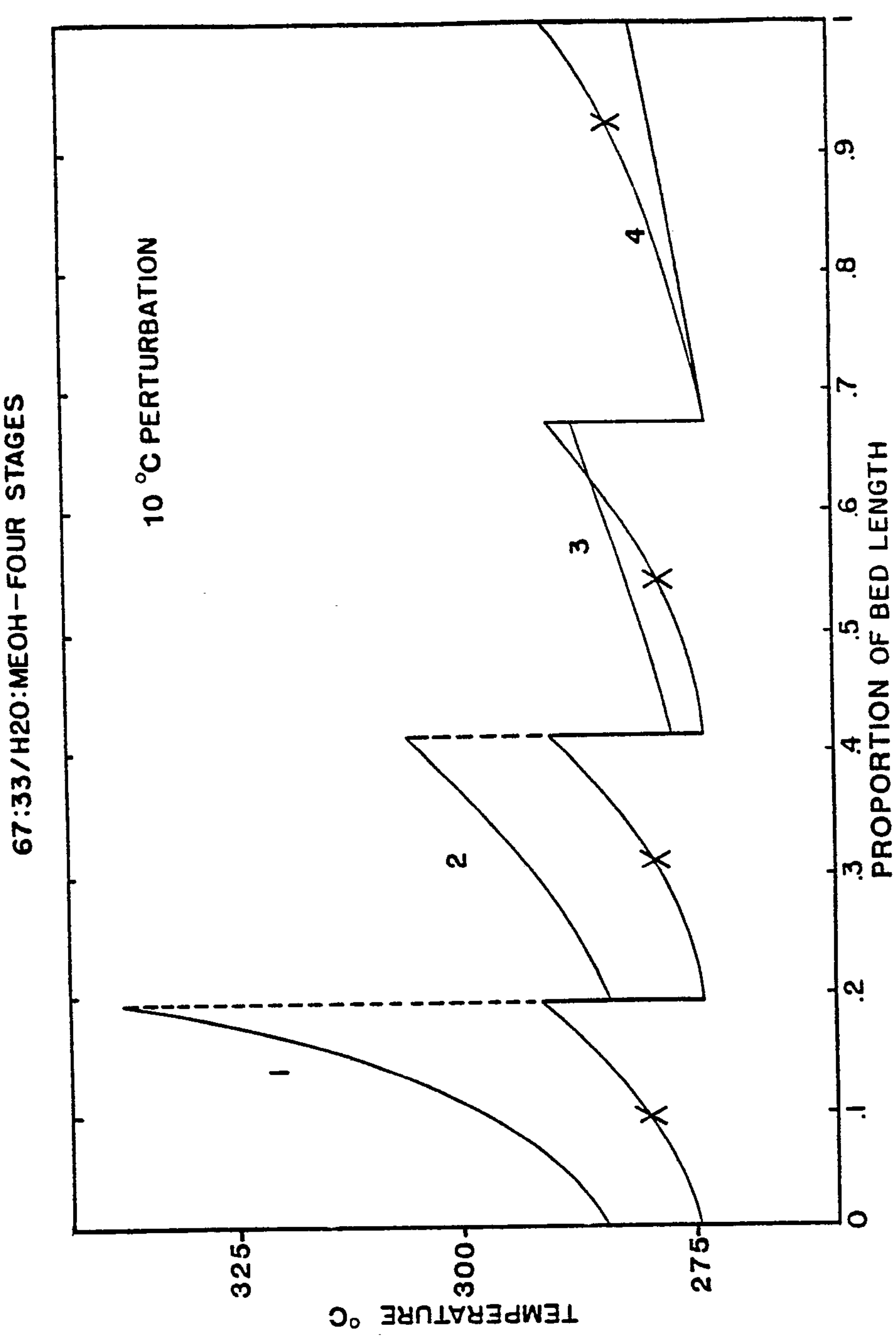

67:33/H2O:MEOH–FOUR STAGES
10 °C PERTURBATION
TEMPERATURE °C
325
300
275
0
.1
.2
.3
.4
.5
.6
.7
.8
.9
PROPORTION OF BED LENGTH
1
2
3
4


FIG. 10